(19) Europäisches Patentamt
European Patent Office
Office européen des brevets

(11) Veröffentlichungsnummer: **0 011 669**
**A1**

# (12) EUROPÄISCHE PATENTANMELDUNG

(21) Anmeldenummer: **79101295.8**

(22) Anmeldetag: **30.04.79**

(51) Int. Cl.$^3$: **C 07 C 49/547**
C 07 C 69/74, C 07 C 45/46
C 07 C 67/313, C 07 C 61/26
C 07 C 51/29

(30) Priorität: **30.11.78 DE 2851790**

(43) Veröffentlichungstag der Anmeldung:
**11.06.80 Patentblatt 80 12**

(84) Benannte Vertragsstaaten:
**BE CH DE FR GB IT NL**

(71) Anmelder: **BAYER AG**
**Zentralbereich Patente, Marken und Lizenzen**
**D-5090 Leverkusen 1, Bayerwerk(DE)**

(72) Erfinder: **Vogel, Emanuel, Prof. Dr.**
**Raschdorffstrasse 7**
**D-5000 Köln 41(DE)**

(72) Erfinder: **Schäfer-Ridder, Maria, Dr.**
**Broichweg 22**
**D-5014 Kerpen(DE)**

(72) Erfinder: **Wagner, Arwed, Dipl-Chem.**
**Bonnstrasse 528**
**D-5030 Hürth(DE)**

## (54) Neue Cycloheptatrien-Verbindungen und Verfahren zur Herstellung von Cycloheptatrien-Verbindungen.

(57) Cycloheptatrien-Verbindungen der Formel

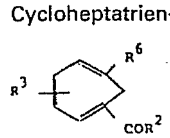

in der $R^2$: Alkyl oder Cycloalkyl bedeutet,
$R^3$: Wasserstoff, Alkyl oder Cycloalkyl und
$R^6$: $COR^2$ oder $COOR^2$ bedeutet und für den Fall, dass $R^3$ kein Wasserstoff oder $R^2$ kein Methyl ist, auch Wasserstoff sein kann.

Verfahren zur Herstellung von acylierten Cycloheptatrien-Verbindungen durch Umsetzung von Cycloheptatrien-Verbindungen mit Acylierungsmitteln in Gegenwart von Acylierungskatalysatoren und weiterhin die Umsetzung von Acetyl-substituierten Cycloheptatrien-Verbindungen mit Hypohalogenit.

EP 0 011 669 A1

BAYER AKTIENGESELLSCHAFT
Zentralbereich
Patente, Marken und Lizenzen

5090 Leverkusen, Bayerwerk
Ha-Th -Kü

Neue Cycloheptatrien-Verbindungen und Verfahren zur Herstellung von Cycloheptatrien-Verbindungen

Die vorliegende Erfindung betrifft neue Cycloheptatrienyl-Verbindungen und ein Verfahren zur Herstellung von Cycloheptatrien-Verbindungen.

Es ist bekannt, durch eine Ringerweiterungsreaktion aus 1-Chlormethyl-3,5-cyclohexadien-1,2-dicarbonsäure-dimethylester durch Umsetzung mit Kalium-tert.-butylat zum Cycloheptatrien-1,6-dicarbonsäure-dimethylester zu gelangen (Helv. Chim. Acta 46, 2893 (1963).

Weiterhin ist die Einführung der Acetylgruppe (Chem. Commun. 1969, 1273) am Cycloheptatrien-eisen-tricarbonyl-Komplex und die Benzoylierung von Cycloheptatrien beschrieben (Chem.Commun. 1969, 1506) worden.

Es wurde ein Verfahren zur Herstellung von Cycloheptatrien-Verbindungen gefunden,das dadurch gekennzeichnet ist, daß man Cycloheptatrien-Derivate der Formel (I)

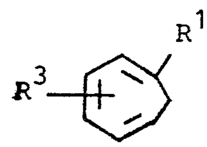

(I),

in der

$R^1$ Wasserstoff, $-COR^2$ oder $-COOR^2$ ist, wobei $R^2$ Alkyl oder Cycloalkyl bedeutet, und

$R^3$ für Wasserstoff, Alkyl oder Cycloalkyl steht,

mit einem Acylierungsmittel der Formel (II)

$$R^2\text{-CO-X} \qquad \text{(II)},$$

in der

$R^2$ die oben angegebene Bedeutung hat und

X für ein Halogen oder $-OCOR^2$ steht,

gegebenenfalls in einem inerten Lösungsmittel, im Temperaturbereich von -60°C bis +60°C in Gegenwart eines Acylierungs-Katalysators umsetzt und gegebenenfalls, wenn $R^1$ Wasserstoff oder $COCH_3$ und $R^2$ Methyl bedeutet, weiter mit einem Hypohalogenit im Temperaturbereich von -10 bis +70°C in wäßriger Lösung, gegebenenfalls unter Mitverwendung eines wasserlöslichen organischen Lösungsmittels, umsetzt.

Die erfindungsgemäß herstellbaren Verbindungen können durch folgende Formel (III)

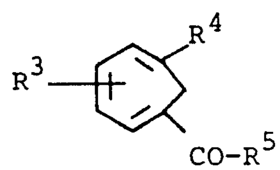

(III),

in der

$R^4$ für Wasserstoff, $COR^2$, $COOR^2$ oder COOH steht,
$R^5$ für $R^2$ oder Hydroxyl steht und
$R^2$ und $R^3$ die oben angegebene Bedeutung haben,

beschrieben werden.

Als Alkyl seien beispielsweise geradkettige oder verzweigte Kohlenwasserstoffreste mit 1 bis 4 Kohlenstoffatomen genannt, wie Methyl, Äthyl, Propyl, Isopropyl, Butyl oder Isobutyl. Bevorzugtes Alkyl ist Methyl oder Äthyl, ganz bevorzugt ist der Methylrest.

Als Cycloalkyl seien beispielsweise ringförmige, gegebenenfalls Methyl-substituierte Kohlenwasserstoffreste mit 4 bis 6 Kohlenstoffatomen im Ring genannt, wie Cyclobutyl, Methyl-cyclobutyl, Cyclopentyl, Methyl-cyclopentyl, Cyclohexyl oder Methyl-cyclohexyl. Bevorzugtes Cycloalkyl ist Cyclopentyl oder Cyclohexyl.

Als Halogen seien beispielsweise Chlor oder Brom, bevorzugt Chlor, genannt.

Acylierungsmittel, die im erfindungsgemäßen Verfahren einsetzbar sind, können sich beispielsweise von niederen verzweigten oder unverzweigten aliphatischen oder cycloaliphatischen Carbonsäuren ableiten.

Als Acylierungsmittel seien beispielsweise die Säurehalogenide oder die Säureanhydride genannt. Als Säurehalogenide seien beispielsweise die Säurechloride oder die Säurebromide, bevorzugt jedoch die Säurechloride, genannt.

Beispielsweise seien als Acylierungsmittel genannt: Acetylchlorid, Acetylbromid, Acetanhydrid, Propionylchlorid, Propionylbromid, Propionsäureanhydrid, Butyrylchlorid, Butyrylbromid, Buttersäureanhydrid, Isobutyrylchlorid, Valeroylchlorid, Cyclobutyrylchlorid, Cyclopentylcarbonsäurechlorid, Cyclohexylcarbonsäurechlorid, Methyl-cyclohexylcarbonsäurechlorid.

Als Acylierungs-Katalysatoren seien beispielsweise Halogenide von Metallen oder Nichtmetallen genannt, wie Aluminiumchlorid, Aluminiumbromid, Zinkchlorid, Titantetrachlorid, Zinntetrachlorid, Eisen(III)-chlorid, Eisen(III)-bromid oder Bortrifluorid. Bevorzugt ist der Einsatz von Aluminiumchlorid oder Zinkchlorid.

Die Acylierung im erfindungsgemäßen Verfahren kann mit oder ohne Lösungsmittel durchgeführt werden. Die Arbeitsweise ohne Lösungsmittel ist zum Beispiel dann möglich, wenn alle Reaktionspartner flüssig sind oder wenn ein flüssiger Reaktionspartner im Überschuß angewendet wird und als Lösungsmittel für die anderen Reaktionspartner dient.

Die Arbeitsweise mit Lösungsmittel kann in den Fällen vorteilhaft sein, in denen alle Reaktionspartner zu einer homogenen Phase gelöst werden sollen oder in Fällen, in denen bei stark exothermer Reaktion zur besseren Wärmeabfuhr ein Verdünnungsmittel benötigt wird.

Als gegebenenfalls einzusetzende Lösungsmittel seien beispielsweise erwähnt: Methylenchlorid, Dichloräthan, Schwefelkohlenstoff oder Nitrobenzol.

Die Acylierung im erfindungsgemäßen Verfahren kann beispielsweise im Temperaturbereich von -60°C bis +60°C, bevorzugt im Bereich von -50°C bis +50°C, durchgeführt werden.

Als Hypohalogenite für die erfindungsgemäße Umsetzung seien beispielsweise Natriumhypochlorit, Natriumhypobromit, Natriumhypojodit, Kaliumhypochlorit, Kaliumhypobromit, Kaliumhypojodit, Calciumhypochlorit, Calciumhypobromit oder Calciumhypojodit benannt. Bevorzugt werden Natriumhypobromit oder Kaliumhypobromit eingesetzt.

Als gegebenenfalls neben dem Wasser einzusetzende organische Lösungsmittel seien solche genannt, die wasserlöslich sind und unter den Reaktionsbedingungen oxydationsstabil sind, beispielsweise Dioxan und Tetrahydrofuran.

Die gegebenenfalls mit Hypohalogenit durchgeführte Umsetzung im erfindungsgemäßen Verfahren kann beispielsweise im Temperaturbereich von -10 bis +70°C, bevorzugt im Bereich von -10° bis +40°C, durchgeführt werden.

Das erfindungsgemäße Verfahren wird gewöhnlich unter Normaldruck ausgeführt. Es kann jedoch bei Anwendung niedrig siedender Lösungsmittel auch unter erhöhtem Druck, beispielsweise bis zu 10 bar, bevorzugt unter dem Eigendruck des Lösungsmittels bei der gewählten Reaktionstemperatur, gearbeitet werden. Mit Hilfe des erfindungsgemäßen Verfahrens können die Cycloheptatrien-carbonyl-Verbindungen der Formel (III) hergestellt werden.

Cycloheptatrien-Verbindungen der Formel (IV)

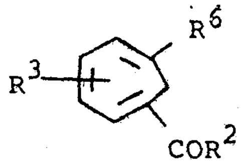

(IV),

in der

$R^2$ Alkyl oder Cycloalkyl bedeutet,
$R^3$ für Wasserstoff, Alkyl oder Cycloalkyl steht und
$R^6$ $COR^2$ oder $COOR^2$ bedeutet und weiterhin für den Fall, daß $R^3$ kein Wasserstoff oder $R^2$ kein Methyl ist, auch Wasserstoff sein kann,

die im erfindungsgemäßen Verfahren hergestellt werden können, sind neu.

Unter den neuen Verbindungen der Formel (IV) seien bevorzugt solche genannt, bei denen $R^2$ Methyl oder Äthyl bedeutet, $R^3$ für Wasserstoff steht und $R^6$ $COCH_3$, $COC_2H_5$, $COOCH_3$ oder $COOC_2H_5$ bedeutet.

Unter den neuen Verbindungen der Formel (IV) seien besonders bevorzugt solche genannt, bei denen $R^2$ Methyl ist, $R^3$ für Wasserstoff steht und $R^6$ $COCH_3$ oder $COOCH_3$ bedeutet.

Die vorliegende Erfindung betrifft somit weiterhin die neuen Cycloheptatrien-Verbindungen der Formel (IV).

Als neue Verbindungen der Formeln (III) und (IV) seien beispielsweise genannt: 1,6-Diacetyl-cyclohepta-1,3,5-trien, 6-Acetyl-cyclohepta-1,3,5-trien-1-carbonsäuremethylester, 1,6-Dipropionylcyclohepta-1,3,5-trien, 1-Acetyl-6-propionyl-cyclohepta-1,3,5-trien, 6-Acetyl-cyclohepta-1,3,5-trien-1-carbonsäure-äthylester, 6-Acetyl-cyclohepta-1,3,5-trien-carbonsäurecyclohexylester, 1-Acetyl-6-cyclohexanoyl-cyclohepta-1,3,5-trien, 6-Acetyl-2-methyl-cyclohepta-1,3,5-carbonsäurebutylester, 6-Acetyl-3-tert.-butyl-cyclohepta-1,3,5-trien-6-carbonsäure-isopropylester, Cyclohepta-1,3,5-trien-1-carbonsäure, Cyclohepta-1,3,5-trien-1,6-dicarbonsäure, Cyclohepta-1,3,5-trien-6-propionyl-1-carbonsäure, Cyclohepta-1,3,5-trien-1,6-dicarbonsäure-monoäthylester, 3-Cyclohexyl-cyclohepta-1,3,5-trien-1-carbonsäure, 4-Methyl-cyclohepta-1,3,5-trien-1,6-dicarbonsäure.

Die Acylierung im erfindungsgemäßen Verfahren sei am Beispiel der Umsetzung von 1-Acetyl-cycloheptatrien mit Acetylchlorid in Gegenwart von Aluminiumchlorid zu 1,6-Diacetyl-cycloheptatrien durch die folgende Formelgleichung erläutert:

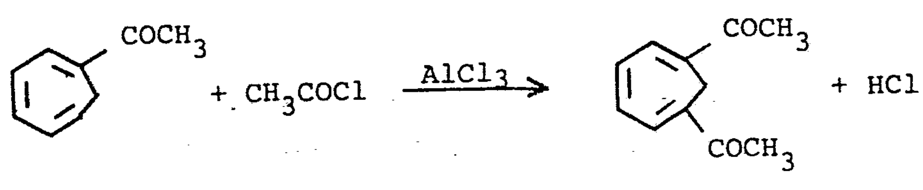

Im allgemeinen wird die Acylierung im erfindungsgemäßen Verfahren wie folgt durchgeführt:

In einer Rührapparatur mit Thermometer und Rückflußkühler werden das Acylierungsmittel und der Katalysator, sowie gegebenenfalls das Lösungsmittel vorgelegt und bei der gewählten Reaktionstemperatur das Cycloheptatrien-Derivat zugetropft. Danach wird das Reaktionsgemisch noch für etwa 1 bis 4 Stunden nachgerührt und anschließend zur Zerstörung des Katalysators unter Kühlung hydrolysiert. Aus dem hydrolysierten Reaktionsgemisch kann das gewünschte Endprodukt durch übliche Aufarbeitungsmethoden gewonnen werden. Hierzu seien beispielsweise die Filtration, die Extraktion der wässrigen Phase mit einem geeigneten Lösungsmittel sowie eine anschließende Umkristallisation, Destillation oder eine Reinigung durch Chromatographieren genannt.

Im allgemeinen wird die gegebenenfalls durchgeführte Umsetzung mit Hypohalogenit wie folgt ausgeführt:

Durch Zugabe von freiem Halogen zu überschüssiger wäßriger Natronlauge bei etwa -10°C wird eine Hypohalogenit-Lösung bereitet. Zu dieser Lösung wird die erfindungsgemäß durch Acylierung erhaltene Cycloheptatrien-Verbindung, gegebenenfalls gelöst in einem wasserlöslichen und oxidationsstabilen organischen Lösungsmittel, innerhalb von 30 Minuten bis 1 Stunde zugetropft. Die Temperatur wird hierbei bei etwa -10°C gehalten. Anschließend läßt man das Reaktionsgemisch 2 bis 20 Stunden nachrühren, wobei es sich auf Raumtemperatur erwärmt. Nach Beendigung der

Reaktion wird die wäßrige Lösung der Carbonsäure von dem entstandenen Haloform getrennt und die Cycloheptatrien-carbonsäure wird durch Ansäuern, beispielsweise mit konzentrierter Salzsäure, ausgefällt.

Das erfindungsgemäße Verfahren gestattet die Herstellung von neuartigen Cycloheptatrien-Derivaten im größeren Maßstab und mit einfacher zugänglichen Chemikalien,als es durch den Stand der Technik möglich war. Es gestattet ferner die Herstellung von Derivaten am nicht durch Eisentricarbonyl-Substituenten komplexierten Cycloheptatrien.

Es ist überraschend, daß die erfindungsgemäßen Acylierungsreaktionen unter Bedingungen, wie sie für aromatische Verbindungen gelten und wie sie bisher nur teilweise an Eisentricarbonyl-substituierten Cycloheptatrienen angewendet werden konnten, in glatter Weise auf Cycloheptatrien-Derivate angewendet werden können.

Es ist ferner überraschend, daß das Cycloheptatrien-System mit seinen drei Doppelbindungen im erfindungsgemäßen Verfahren nicht verändert wird. Weiterhin ist es überraschend, daß die Acylierung nur in der Nachbarschaft der Methylengruppe eintritt und die übrigen denkbaren Isomeren nicht gebildet werden.

Es ist überraschend, daß bei Umsetzung des 1-Acetylcycloheptatriens mit Hypohalogenit zur entsprechenden Carbonsäure die Doppelbindungen des Cycloheptatriens unverändert bleiben.

Die Cycloheptatrien-Derivate, die durch das erfindungsgemäße Verfahren hergestellt werden können, sind Zwischenprodukte für Geruchsstoffe und Aromen, für Insektizide und Herbizide.

Sie sind ferner Monomere und Comonomere für Polykondensate und Polymerisate mit wertvollen Eigenschaften. So ergibt beispielsweise die erfindungsgemäße Oxidation des 1,6-Diacetyl-cycloheptatriens zur Cycloheptatrien-1,6-dicarbonsäure ein wertvolles Monomeres oder Comonomeres für Polykondensate, wie beispielsweise für Polyester oder Polyamide, und für Carboxylgruppen-haltige Polymerisate, die gegebenenfalls weiter vernetzt werden können, in einer dem Fachmann bekannten Weise.

Beispiel 1

1-Acetylcyclohepta-1,3,5-trien

Zu 471 g (6 Mol) Acetylchlorid in 900 ml $CH_2Cl_2$ gibt man bei Raumtemperatur eine Katalysatorlösung, bestehend aus 546 g (4 Mol) wasserfreiem $ZnCl_2$ und 400 ml Eisessig. Zu dieser Lösung tropft man unter Rühren bei -50°C 460 g (5 Mol) frisch destilliertes Cycloheptatrien hinzu und läßt 4 Stunden bei -30°C nachrühren. Anschließend wird das Reaktionsgemisch mit 2 l Eiswasser hydrolysiert und extrahiert. Die wäßrige Phase wird noch zweimal mit $CH_2Cl_2$ gewaschen. Die vereinigten organischen Phasen wäscht man 1 mal mit wäßriger Ammoniaklösung und 1 mal mit Wasser. Nach Trocknen über $MgSO_4$ und Abziehen des Lösungsmittels wird der Rückstand im Ölpumpenvakuum destilliert ($Kp_{0,1}$ = 43°C). Ausbeute: 311 g (46 % der theoretischen Ausbeute) an 1-Acetylcyclohepta-1,3,5-trien.

Beispiel 2

1,6-Diacetylcyclohepta-1,3,5-trien

In 1,5 l $CH_2Cl_2$ werden 770 g (5,65 Mol) wasserfreies $AlCl_3$ aufgeschlämmt. Dazu gibt man unter Rühren 440 g (5,65 Mol) Acetylchlorid, erhitzt die fast klare Lösung bis zum Rückfluß und tropft 300 g (2,25 Mol) 1-Acetylcyclohepta-1,3,5-trien hinzu. Nach ca. zwei Stunden Erhitzen unter Rückfluß hat sich das gesamte 1-Acetyl-cycloheptatrien umgesetzt (dünnschichtchromatographische Kontrolle). Man kühlt das Reaktionsgemisch auf -20°C ab und hydrolysiert vorsichtig mit 250 ml Eisessig, anschließend mit 1 l Wasser. Das hydrolysierte Reaktionsgemisch wird mit 2 bis 3 l Eiswasser extrahiert. Die wäßrige Phase wird noch 2 mal mit $CH_2Cl_2$ gewaschen. Die vereinigten organischen Phasen wäscht man 1 mal mit Wasser. Nach Abdestillieren des Lösungsmittels erhält man 295,5 g (= 75 % der theoretischen Ausbeute) an rohem 1,6-Diacetyl-cyclohepta-1,3,5-trien. Sauberes Produkt erhält man durch Destillation ($Kp_{0,4-0,5}$: 120 bis 125°C) und anschließende Umkristallisation aus Pentan (Fp. = 44 bis 45°C). Die schwach gelben Kristalle sind lange Zeit im Dunkeln haltbar; Lichteinwirkung verfärbt sie braun. C,H-Analyse: Ber. C 74,97 %, H 6,68 %, Gef. 75,01 %, 6,90 %.

Beispiel 3

6-Acetyl-cyclohepta-1,3,5-trien-1-carbonsäuremethylester

251 g (1,9 Mol) $AlCl_3$ werden bei 0°C in 600 ml Methylenchlorid unter Feuchtigkeitsausschluß suspendiert. Bei 0°C werden 135 g (1,75 Mol) Acetylchlorid zugetropft. Nach kurzem Rühren bei Raumtemperatur wird das Gemisch zum Sieden erhitzt, und 90 g (0,6 Mol) Cyclohepta-1,3,5-trien-1-carbonsäuremethylester werden im Verlauf von 30 Minuten nuten zugetropft. Nach einstündigem Erhitzen am Rückfluß

wird bei -15°C bis -10°C mit 200 ml verdünnter Essigsäure, anschließend mit 600 ml Wasser, vorsichtig hydrolysiert. Die Methylenchloridphase wird abgetrennt, die wäßrige Phase zweimal mit Methylenchlorid extrahiert. Nach Waschen der vereinigten organischen Phasen mit Wasser, $NaHCO_3$-Lösung (starke $CO_2$-Entwicklung!) und Wasser, Trocknen mit $MgSO_4$ und Entfernen des Lösungsmittels wird der Rückstand destilliert. Man erhält 80,6 g Acetylester als gelbes Öl in 70 % der theoretischen Ausbeute (gelbe Nadeln vom Fp.= 47°C, $Kp_{0,1}$= 110°C; C,H-Analyse: Ber. C 68,74, H 6,29, Gef. C 68,89, H 6,16).

Beispiel 4

Cyclohepta-1,3,5-trien-1,6-dicarbonsäure

In wäßrige Natronlauge (900 g (22,5 Mol) in 3 l Wasser) tropft man bei -10°C 1,08 kg (6,75 Mol) Brom. Zu dieser Lösung werden innerhalb von 30 Minuten 197 g (1,125 Mol) rohes 1,6-Diacetylcyclohepta-1,3,5-trien, gelöst in 100 ml Dioxan, zugegeben. Dabei sollte die Temperatur bei -10°C gehalten werden. Das Reaktionsgemisch läßt man während 2 Stunden auf 20°C auftauen und rührt bei dieser Temperatur noch 30 Minuten nach. Anschließend wird in einem Scheidetrichter die Phasentrennung durchgeführt, wobei die Temperatur durch Wasserkühlung auf ca. 20°C gehalten wird.

Die wäßrige Phase wird noch einmal mit $CH_2Cl_2$ gewaschen. Das überschüssige NaOBr reduziert man mit 1 l 25 %iger

$Na_2S_2O_5$-Lösung. Nach Ansäuern mit konzentrierter Salzsäure fällt feinkristalline Cycloheptatrien-1,6-dicarbonsäure an. Sie wird abgesaugt, mit Wasser neutral gewaschen und bei 70°C bis zur Gewichtskonstanz getrocknet.
Ausbeute: 95 g (47 % der theoretischen Ausbeute).

Beispiel 5

Cyclohepta-1,3,5-trien-carbonsäure

Zu einer Lösung von 300 g (7,5 Mol) NaOH in 1000 ml Wasser werden 341 g (2,13 Mol) $Br_2$ bei -10°C zugetropft, anschließend bei -10°C 100 g (0,75 Mol) 1-Acetylcycloheptatrien. Nach sechsstündigem Rühren bei 0°C wird das Reaktionsgemisch über Nacht unter Eiskühlung gerührt. Nach Abtrennen der wäßrigen Phase vom gebildeten Bromoform versetzt man diese mit einer Lösung von 75 g $Na_2S_2O_5$ in 100 ml Wasser und säuert mit konzentrierter Salzsäure unter Kühlung an ($SO_2$-Entwicklung!). Die ausgefallene Säure wird abgesaugt, mit Wasser gewaschen und getrocknet (Fp. = 64°C, Ausbeute 81,2 g = 80 % der theoretischen Ausbeute).

Beispiel 6

6-Acetyl-cyclohepta-1,3,5-trien-1-carbonsäureäthylester

Analog Beispiel 3 wurde der Äthylester umgesetzt. 6-Acetyl-cyclohepta-1,3,5-trien-1-carbonsäureäthylester wurde in 73 % der theoretischen Ausbeute erhalten. $Kp._{0,1}$: 114°C.

Patentansprüche

1. Cycloheptatrien-Verbindungen der Formel

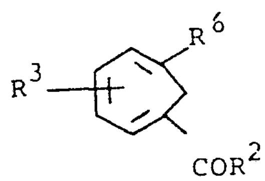

in der

$R^2$ Alkyl oder Cycloalkyl bedeutet,

$R^3$ für Wasserstoff, Alkyl oder Cycloalkyl steht und

$R^6$ $-COR^2$ oder $-COOR^2$ bedeutet und weiterhin für den Fall, daß $R^3$ kein Wasserstoff oder $R^2$ kein Methyl ist, auch Wasserstoff sein kann.

2. Cycloheptatrien-Verbindungen nach Anspruch 1, dadurch gekennzeichnet, daß $R^2$ Methyl oder Äthyl bedeutet, $R^3$ für Wasserstoff steht und $R^6$ $COCH_3$, $COC_2H_5$, $COOCH_3$ oder $COOC_2H_5$ bedeutet.

3. Verfahren zur Herstellung von Cycloheptatrien-Verbindungen, dadurch gekennzeichnet, daß man Cycloheptatrien-Derivate der Formel

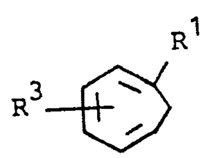

,

in der

$R^1$ Wasserstoff, $-COR^2$ oder $-COOR^2$ ist, wobei $R^2$ Alkyl oder Cycloalkyl bedeutet, und
$R^3$ für Wasserstoff, Alkyl oder Cycloalkyl steht,

mit einen Acylierungsmittel der Formel

$$R^2\text{-CO-X} \ ,$$

in der

$R^2$ die oben angegebene Bedeutung hat und
X für ein Halogen oder $-OCOR^2$ steht,

gegebenenfalls in einem inerten Lösungsmittel, im Temperaturbereich von $-60^{\circ}C$ bis $+60^{\circ}C$ in Gegenwart eines Acylierungs-Katalysators umsetzt und gegebenenfalls, wenn $R^1$ Wasserstoff oder $COCH_3$ und $R^2$ Methyl bedeutet, weiter mit einem Hypohalogenit im Temperaturbereich von -10 bis $+70^{\circ}C$ in wäßriger Lösung, gegebenenfalls unter Mitverwendung eines wasserlöslichen organischen Lösungsmittels, umsetzt.

4. Verfahren nach Anspruch 3, dadurch gekennzeichnet, daß das Acylierungsmittel ein Säurechlorid ist.

Europäisches
Patentamt

## ☐ GEBÜHRENPFLICHTIGE PATENTANSPRÜCHE

Die vorliegende europäische Patentanmeldung enthielt bei ihrer Einreichung mehr als zehn Patentansprüche.

☐ Alle Anspruchsgebühren wurden innerhalb der vorgeschriebenen Frist entrichtet. Der vorliegende europäische Recherchenbericht wurde für alle Patentansprüche erstellt.

## ☒ MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung; sie enthält mehrere Erfindungen oder Gruppen von Erfindungen, nämlich:

1. Patentansprüche 1,2,3 (teilweise) und 4 (weil referierend nach 3): Cycloheptatrien-Verbindungen gemäss Patentanspruch 1 und ihre Herstellung durch Acylierung.

2. Patentansprüche 3 (teilweise) und 4 (weil referierend nach 3): Herstellung von Cycloheptatrien-Verbindungen nicht gehörend zu den Verbindungen gemäss Patentanspruch 1 durch Acylierung (insbesonderes Verbindungen der Formel

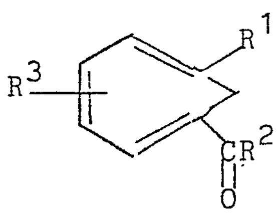

in der .

$R^1$ und $R^3$ Wasserstoff bedeuten oder $R^1$ Wasserstoff und $R^2$ Methyl bedeuten)

3. Patentansprüche 3 (teilweise) und 4 (weil referierend nach 3) Herstellung von Cycloheptatrien-carbonsäuren (siehe Seite 10, Zeilen 1-4) durch Acylierung und durch Umsetzung von einer beschränkten Klasse von acylierten Cycloheptatrien-Verbindungen (d.h. nur wenn $R^1$ Wasserstoff oder $COCH_3$ und $R^2$ Methyl bedeutet) mit einem Hypohalogenit.

☐ Alle weiteren Recherchengebühren wurden innerhalb der gesetzten Frist entrichtet. Der vorliegende europäische Recherchenbericht wurde für alle Patentansprüche erstellt.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind,

nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen,

nämlich Patentansprüche: 1,2,3 und 4 in sofern gemäss ...

0011669

Europäisches Patentamt

# EUROPÄISCHER RECHERCHENBERICHT

Nummer der Anmeldung
EP 79 10 1295

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| X | JOURNAL OF THE CHEMICAL SOCIETY (C)(1971, Seiten 1592-96 London, GB, J.A. BLAIR et al.: "The reaction of cycloheptatriene with acyl halides in the presence of lewis acids. A convenient synthesis of 1-acyl-cycloheptatrienes" ⁎ Seite 1596 ⁎ -- | 1-4 | C 07 C 49/547<br>69/74<br>45/46<br>67/313<br>61/26<br>51/29 |
| D | HELVETICA CHIMICA ACTA, 1963, Vol. XLVI, Fasciculus VII, Nr. 320-321, Seiten 2893-2905 Basel, CH, R. DARMS et al.: "Cycloheptatrien-1,6 Dicarbonsäure und Bicyclo-(4,1,0)-2,4-heptadien-1,6-dicarbonsäureanhydrid" ⁎ Seite 2896 ⁎. ---- | 1 | RECHERCHIERTE SACHGEBIETE (Int. Cl.³)<br>C 07 C 49/61<br>69/95<br>45/14<br>67/00 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

~~Der vorliegende Recherchenbericht wurde für alle Patentansprüche erstellt.~~

| Recherchenort | Abschlußdatum der Recherche | Prüfer |
|---|---|---|
| Den Haag | 30-10-1979 | BONNEVALLE |

PA form 1503.1 06.78